# DEMANDE DE BREVET EUROPEEN

(11) **EP 0 819 419 A1**
(43) Date de publication de la demande: **21.01.1998**
(21) Numéro de dépôt: 97401457.3
(22) Date de dépôt: 23.06.1997
(51) Int. Cl.: A61K 7/027, A61K 7/48

(54) **Composition comprenant un composant hydrophile, et l'association d'un composé volatil et d'une huile siliconée phénylée, et utilisation de ladite association**

(30) Priorité: 17.07.1996 FR 9608956
(71) Demandeur: L'OREAL, 75008 Paris (FR)
(72) Inventeur: Agostini, Isabelle, 92290 Chatenay Malabry (FR); Miguel Colombel, Dolorès, 94240 L'Hay les Roses (FR)
(74) Mandataire: Dodin, Catherine

(57) **Abrégé**

L'invention concerne une composition cosmétique, dermatologique, hygiénique et/ou pharmaceutique, comprenant un composant hydrophile et une phase grasse qui comprend au moins un composé volatil et au moins une huile siliconée phénylée. Ladite composition est notamment une composition ayant une teneur améliorée.

L'invention concerne également l'utilisation de l'association d'un composé volatil et d'une huile siliconée phénylée afin de limiter le transfert et/ou la migration, d'une telle composition cosmétique, dermatologique, hygiénique et/ou pharmaceutique comprenant un composant hydrophile.

## Description

La présente invention a pour objet une composition notamment cosmétique pouvant se présenter sous forme d'un stick ou d'une pâte souple, et susceptible d'être utilisée pour le soin et/ou le maquillage de la peau, des semi-muqueuses et/ou des muqueuses, et en particulier des lèvres du visage.

Les compositions cosmétiques ou pharmaceutiques grasses solides habituellement utilisées notamment sous forme de bâtons, par exemple de rouge à lèvres, présentent l'inconvénient d'être peu hydratantes. Pour pallier cet inconvénient, on a tenté d'y introduire de l'eau en préparant des produits émulsionnés.
On peut à cet égard citer la demande de brevet français FR2237615 qui décrit une émulsion eau-dans-huile contenant de 1 à 50 % en poids d'eau. Toutefois, ce type de composition pose des problèmes d'évaporation de l'eau. La composition décrite dans cette demande de brevet contient également de 1 à 10 % d'un composé polyhydroxylé afin d'obtenir une répartition uniforme des substances colorantes dans l'émulsion eau-dans-huile.
Le problème de l'évaporation de l'eau des dispersions solides a été résolu selon la demande de brevet européen EP374332 par l'incorporation d'une huile siliconée ainsi que d'un organopolysiloxane modifié par un polyoxyalkylène. Les compositions ainsi modifiées peuvent également contenir des polyols en faible quantité en tant qu'humectants additionnels.
Dans la demande de brevet japonais JP01-143812, il est décrit l'incorporation d'une quantité importante d'alcools polyvalents dans une émulsion solide à usage cosmétique par le biais d'une huile de silicone et d'un organopolysiloxane de polyoxyalkylène dénaturé. Or, avec ce système, il est difficile d'obtenir une bonne homogénéité de la formule.
On connaît enfin la demande EP524892 qui a pour but de pallier les inconvénients décrits ci-dessus, en proposant un procédé particulier permettant d'obtenir une dispersion solide et anhydre qui comprend des corps gras et un alcool polyhydrique en quantité importante.

Toutefois, on a constaté que toutes ces compositions, lorsqu'elles sont appliquées sur la peau, les muqueuses ou les semi-muqueuses, présentent l'inconvénient de transférer. On entend par là que la composition est susceptible de se déposer, au moins en partie, sur certains supports avec lesquels elle est mise en contact, tels que, par exemple, un verre, un vêtement ou la peau. En se déposant, ladite composition laisse une trace sur ledit support. ll s'en suit donc une persistance médiocre de la composition sur la peau ou les muqueuses, d'où la nécessité de renouveler régulièrement son application.
Un autre inconvénient de ces compositions réside dans le problème de migration. On a en effet constaté que certaines compositions avaient tendance à se propager à l'intérieur des ridules et/ou des rides de la peau, dans le cas des fonds de teint; dans les ridules qui entourent les lèvres, dans le cas des rouges à lèvres; dans les plis de la paupière, dans le cas des fards à paupières.

On a également constaté, dans le cas notamment des fards à paupières, l'apparition de stries dans le maquillage, générées par les mouvements des paupières. Tous ces phénomènes engendrent un effet inesthétique que l'on souhaite bien évidemment éviter.
Ces inconvénients, qui sont généralement présents pour les compositions de rouges à lèvres usuels, sont encore plus prononcés lorsque la composition comprend des polyols. Ceci est en particulier dû au fait que les polyols sont des composés collants et visqueux, qui ont tendance à adhérer et à se déposer sur le support avec lequel ils sont mis en contact, en y laissant une trace.

Il subsiste donc le besoin d'une composition notamment cosmétique qui transfère peu ou pas du tout, c'est-à-dire d'une composition 'sans transfert', tout en possédant de bonnes propriétés d'hydratation et/ou émollientes.
Notamment il subsiste le besoin d'une composition étant susceptible de comprendre des composés hydrophiles tels que des polyols en grande quantité, tout en conservant une très bonne tenue c'est-à-dire en ne transférant pas, ne tachant pas, ou peu, un support avec lequel elle serait en contact, et en ne migrant pas au cours du temps.
Le but de la présente invention est donc de proposer une telle composition, qui ne transfère pas, ou peu, bien qu'elle comprenne des composés hydrophiles.

L'invention a donc pour objet une composition cosmétique, dermatologique, hygiénique et/ou pharmaceutique, comprenant un composant hydrophile et une phase grasse qui comprend au moins un composé volatil et au moins une huile siliconée phénylée.
L'invention a également pour objet l'utilisation de l'association d'un composé volatil et d'une huile siliconée phénylée afin de limiter, diminuer et/ou supprimer le transfert et/ou la migration, et/ou afin d'améliorer la tenue, d'une composition cosmétique, dermatologique, hygiénique et/ou pharmaceutique comprenant un composant hydrophile.
L'invention a encore pour objet un procédé pour limiter, diminuer et/ou supprimer le transfert d'une composition cosmétique, dermatologique, hygiénique et/ou pharmaceutique comprenant un composant hydrophile, notamment une composition de maquillage et/ou de soin de la peau, des muqueuses, des semi-muqueuses et/ou des phanères, consistant à introduire dans ladite composition au moins un composé volatil et au moins une huile siliconée phénylée.

On a constaté que la composition selon l'invention est facilement applicable sur la peau, et qu'elle permet entre autre un maquillage de texture légère, qui reste confortable à porter tout au long de la journée. De plus, la composition selon l'invention possède d'excellentes propriétés émollientes par application sur la peau.

La composition selon l'invention trouve en particulier une application particulièrement intéressante dans le domaine du soin et/ou du maquillage de la peau, des muqueuses, des semi-muqueuses et des phanères. On entend notamment par muqueuse, la partie interne de la paupière inférieure; parmi les semi-muqueuses, on entend plus particulièrement les lèvres du visage; par phanères, on entend les cils, sourcils, cheveux et ongles. Ainsi, l'invention trouve une application toute particulière dans le domaine des produits de maquillage des lèvres du visage, mais aussi des produits de soin des lèvres ainsi que dans le domaine des produits de maquillage et de soin de la peau tels que les fonds de teint, les autobronzants ou les produits solaires.

La composition selon l'invention comprend donc au moins un composant hydrophile, qui peut être, de préférence, un alcool polyhydrique et/ou éventuellement une phase aqueuse épaissie, voire gélifiée.
L'alcool polyhydrique peut être un composé ayant 2-8 atomes de carbone et 2-6 fonctions hydroxyles, tel que l'éthylène glycol, le glycérol, le propane-1,2 diol, la diglycérine, l'érythritol, l'arabitol, l'adonitol, le sorbitol et le dulcitol. L'alcool polyhydrique peut également être un polyéther alcool de poids moléculaire moyen de 150-600, tel que le polyéthylène glycol 300 et la polyglycérine 500. On peut également employer un mélange d'alcools polyhydriques.
La phase aqueuse épaissie ou gélifiée, peut comprendre de l'eau et/ou une eau florale telle que l'eau de bleuet, et/ou une eau minérale telle que l'eau de VITTEL, l'eau de LUCAS ou l'eau de LA ROCHE POSAY.
Elle peut être épaissie par la présence de tout agent épaississant connu de l'homme du métier. On peut notamment citer :
. les extraits d'algues tels que l'agar-agar, les carraghénanes et les alginates,
. les extraits de graines tels que les extraits de caroube ou de guar,
. les extraits de fruits, notamment la pectine,
. les exsudats de plantes, tels que la gomme arabique, l'adragante, la gomme de karaya et la gomme de ghatty,
. les dérivés cellulosiques, tels que la carboxyméthylcellulose,
. les gélifiant d'origine animale tels que la gélatine ou les caseinates,
. les exsudats de micro-organismes tels que la gomme xanthane,
. les gélifiants synthétiques tels que les dérivés de polymère acrylique (Carbomer, Carbopol, Pemulen) ou les dérivés du silicium (Laponite, Lapomer ou Veegum).
L'agent épaississant peut être présent dans la phase aqueuse à raison de 0,2-10% en poids par rapport à la phase aqueuse.
En outre, le composant hydrophile peut comprendre des additifs hydrosolubles, notamment des vitamines, des actifs cosmétiques et/ou pharmaceutiques, des colorants, des acides aminés, des filtres UV.
Le composant hydrophile peut représenter 4 à 40% en poids de la composition, de préférence 8 à 25% en poids.

De préférence, le composant hydrophile est dispersé dans au moins une partie de la phase grasse, avant d'être mélangé au reste des constituants de la composition. Lorsque le composant hydrophile comprend au moins un alcool polyhydrique, la taille des particules dudit alcool est de préférence inférieure ou égale à un micron.
Afin d'obtenir une dispersion plus fine du composant hydrophile dans la composition, on peut ajouter à ladite composition au moins un agent tensioactif. Cet agent tensioactif peut être anionique ou non ionique, de préférence avec un HLB inférieur à 10, préférentiellement inférieur à 5. On peut citer notamment des dérivés siliconés tels que les tensioactifs siliconés, ou tout autre tensioactif usuel tel que les lécithines, les succinylglycérides, les alkylphosphates, le lanolate de magnésium, et leurs mélanges.

La composition selon l'invention comprend donc en outre une phase grasse, qui comprend notamment des composés volatils. Ces composés volatils peuvent être choisis en particulier parmi les huiles hydrocarbonées et/ou les huiles siliconées, cycliques ou linéaires, seules ou en mélange.
Par composé volatil, on entend dans la présente description, tout composé susceptible de s'évaporer au contact de la peau. De préférence, on utilise des huiles volatiles dont le point éclair est suffisamment élevé pour permettre l'utilisation de ces huiles en formulation, et suffisamment bas pour obtenir l'effet évanescent souhaité. On emploie de préférence des huiles volatiles dont le point éclair est de l'ordre de 40-100°C.
Parmi les huiles siliconées volatiles, on peut citer, seules ou en mélange,
- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane.
- les cyclocopolymères du type diméthylsiloxane/méthylalkylsiloxane, tels que la SILICONE FZ 3109 vendue par la société UNION CARBIDE, qui est un cyclocopolymère diméthylsiloxane/méthyloctylsiloxane.
- les silicones volatiles linéaires ayant de 2 à 9 atomes de silicium. Il s'agit par exemple de l'hexaméthyldisiloxane ou un PDMS de faible viscosité (1 cSt). On peut encore citer les alkyltrisiloxanes tels que l'hexylheptaméthyltrisiloxane ou l'octylheptaméthyltrisiloxane.
Parmi les huiles hydrocarbonées volatiles, on peut citer les isoparaffines. et notamment l'isododécane
La composition selon l'invention peut comprendre 8-70% en poids, de préférence 15-55%, de composés volatils, par rapport au poids total de la composition.

La composition selon l'invention comprend en outre au moins une huile de silicone phénylée, notamment un polyphénylméthylsiloxane ou un phényltriméthicone, ou un mélange de différentes huiles siliconées phénylées.
En particulier, ces huiles peuvent répondre à la formule suivante : 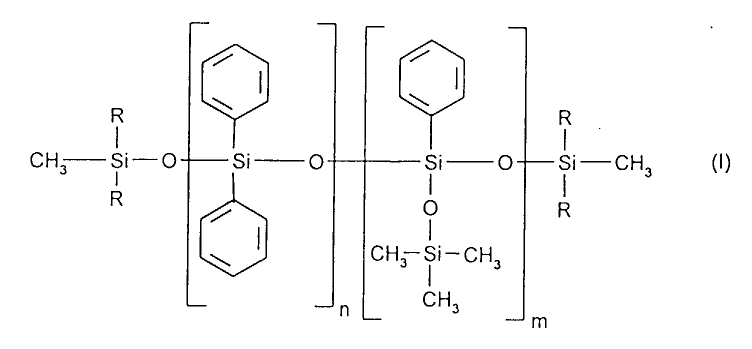 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100.
De préférence, R est un radical méthyle, éthyle, propyle, isopropyle, décyle, dodécyle ou octadécyle, ou encore un radical phényle, tolyle, benzyle ou phénéthyle.
Parmi ces huiles phénylées, on peut citer l'huile Belsil PDM1000 de Wacker, les huiles DC556 ou SF558 de Dow Corning, l'huile Abil AV8853 de Goldschmidt ou l'huile Silbione 70633V30 de Rhône Poulenc.
La composition selon l'invention peut comprendre 1-35% en poids, de préférence 20-30%, d'huiles siliconées phénylées.

La composition selon l'invention peut également comprendre au moins une cire, qui peut assurer notamment la résistance mécanique de la composition, lorsqu'elle se présente sous la forme d'un stick. Lorsqu'elle se présente sous la forme d'une pâte souple ou d'un produit coulé, la composition selon l'invention peut comprendre une quantité moins importante de cire.
On peut employer toute cire connue dans l'art antérieur parmi lesquelles, seules ou en mélange, on peut citer les cires animales, végétales, minérales et synthétiques telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; les cires de silicone; les cires fluorées.
De préférence, les cires entrant dans la composition peuvent présenter un point de fusion supérieur à 45°C environ, et en particulier supérieur à 55°C, et/ou un indice de pénétration de l'aiguille à 25°C de préférence compris entre 3 et 40.
De préférence, la composition comprend 0,5 à 30% en poids de cire, notamment 10 à 20% en poids.

En outre, la composition peut comprendre, en plus des composés cités ci-dessus, les corps gras usuellement utilisés dans le domaine d'application envisagé, tels que des huiles, des gommes et/ou des corps gras pâteux d'origine végétale, minérale, animale, synthétique ou siliconée. Ces corps gras peuvent en particulier être choisis de manière variée par l'homme du métier afin de préparer une composition ayant les propriétés souhaitées, par exemple en consistance ou en texture.
En particulier, on peut citer les polydiméthylsiloxanes (PDMS) et les alkyldiméthicones, ainsi que les silicones modifiées par des groupements aliphatiques et/ou aromatiques, éventuellement fluorés, ou par des groupements fonctionnels tels que des groupements hydroxyles, thiols et/ou amines.
On peut encore citer l'huile de paraffine, de vaseline, le perhydrosqualène, l'huile d'arachide, d'amande douce, de calophyllum, de palme, de ricin, d'avocat, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides.
Toutefois, on a constaté que l'amélioration de la tenue de la composition selon l'invention, ainsi que son absence de migration et/ou de transfert, pouvait être particulièrement intéressante lorsque la composition comprenait moins de 20% en poids d'huile hydrocarbonée non volatile, de préférence moins de 5% en poids, voire pas d'huiles hydrocarbonées non volatiles du tout.

Il est également possible d'ajouter à la composition selon l'invention une quantité suffisante d'un polymère pour la stabiliser davantage en atmosphère humide. De tels polymères doivent être liposolubles et posséder un taux de motifs hydrophiles très faible. Parmi ceux-ci, on peut citer les polyalkylènes (notamment les polyéthylènes et polybutènes), les polyacrylates et les polymères siliconés compatibles avec les corps gras. Parmi les polyalkylènes, on peut citer le polybutène, notamment celui vendu par la société Amoco sous la dénomination Indopol.
Le rapport en poids du polymère à l'alcool polyhydrique est de préférence inférieur à 4, plus particulièrement compris entre 0,25 et 2.

La composition peut comprendre également une phase particulaire, qui peut comprendre des pigments et/ou des nacres et/ou des charges habituellement utilisés dans les compositions cosmétiques. Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition. Par nacres, il faut comprendre des particules irisées qui réfléchissent la lumière, d'origine naturelle ou synthétique.
Les pigments peuvent être présents dans la composition à raison de 0 à 15% en poids de la composition finale, et de préférence à raison de 8 à 10%. Ils peuvent être blancs ou colorés, minéraux et/ou organiques.
On peut citer les dioxydes de titane, de zirconium ou de cérium, ainsi que les oxydes de zinc, de fer ou de chrome, le bleu ferrique, le noir de carbone, les outremers, le pyrophosphate de manganèse et certaines poudres métalliques telles que celles d'argent ou d'aluminium. On peut encore citer les laques couramment employées pour conférer aux lèvres et à la peau un effet de maquillage, qui sont des sels de calcium, de baryum, d'aluminium ou de zirconium, de colorants acides tels que les colorants halogéno-acides, azoïques, anthraquinoniques.
Les nacres peuvent être présentes dans la composition à raison de 0 à 20% en poids, de préférence à un taux de l'ordre de 8 à 15% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Les charges, qui peuvent être présentes à raison de 0 à 30% en poids, de préférence 5 à 15%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères telles que l'Expancel (Nobel Industrie), le polytrap (Dow Corning) et les microbilles de résine de silicone (Tospearls de Toshiba, par exemple).

La composition peut comprendre en outre tout additif usuellement utilisé dans le domaine cosmétique, tel que des antioxydants, des parfums, des huiles essentielles, des conservateurs, des actifs cosmétiques lipophiles et/ou hydrophiles, des hydratants, des vitamines, des acides gras essentiels, des sphingolipides, des agents autobronzants tels que la DHA, des filtres solaires.
Ces additifs se trouvent selon leur solubilité soit dans la phase grasse soit dans la phase comprenant l'alcool polyhydrique ou la phase aqueuse.
Bien entendu l'homme du métier veillera à choisir les éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut également comprendre au moins un agent actif, parmi lesquels on peut citer les agents actifs contre les micro-organismes, notamment à activité antivirale, antibactérienne ou antifongique; les agents à activité anti-inflammatoire ou immunomodulatrice; les agents antagonistes des neuromédiateurs ou modulant le relarguage des neuromédiateurs; les agents modulant la différenciation et/ou la prolifération cellulaire et/ou la pigmentation et/ou régulant la kératinisation; les agents actifs dans le traitement et/ou la prévention des cheilites; les antihistaminiques; les agents cicatrisants.
Selon les pathologies à traiter, il peut être judicieux d'associer plusieurs agents actifs, ou encore de leur adjoindre des actifs secondaires qui vont favoriser la prévention ou le traitement de l'affection, et/ou traiter des symptômes associés à cette affection. Parmi les agents actifs secondaires, on peut citer les anesthésiques locaux, les antiseptiques, les hydratants et/ou émollients, et les filtres solaires notamment chimiques ou minéraux. La composition peut en outre contenir un agent favorisant la solubilisation ou la compatibilité des agents actifs avec l'excipient de la composition, et/ou favorisant la pénétration desdits agents actifs dans la peau ou les muqueuses. Il peut s'agir par exemple du myristate d'isopropyle, de l'acide oléique, de la lécithine ou de certains alcools.

La composition selon l'invention peut se présenter sous la forme d'un stick ou bâton, sous la forme d'un liquide huileux, éventuellement gélifié, ou encore sous la forme d'une pâte souple dont on peut mesurer la viscosité. Ladite viscosité dynamique à 25°C est généralement comprise entre 3 et 35 Pa.s, mesurée avec un viscosimètre rotatif CONTRAVES TV équipé d'un mobile "MS-r4" à la fréquence de 60 Hz.
De préférence, la composition selon l'invention se présente sous forme anhydre.

La composition selon l'invention peut être préparée de manière usuelle par l'homme du métier. En particulier, le composant hydrophile peut être incorporé dans la composition grâce à l'utilisation, pendant l'incorporation, d'une turbine à une vitesse de rotation minimum ce qui produit des particules de très faible taille, notamment inférieure ou égale à 1 micron. On peut ainsi incorporer une quantité importante d'alcool polyhydrique, par exemple.
Notamment, on peut préparer la composition en chauffant tout ou partie des corps gras, à l'exclusion des composés volatils, et le composant hydrophile à une température comprise entre 65 et 95°C, puis en mélangeant à l'aide d'une turbine ayant une vitesse de rotation d'au moins 1500 tours par minute, de préférence de 2500-3500 tr/min.
On ajoute alors les composés volatils et les éventuels additifs susceptibles de craindre la chaleur. L'émulsion ainsi obtenue est coulée dans un moule approprié. La turbine utilisable dans le procédé selon l'invention peut être de tout modèle connu dans le commerce pour la préparation des compositions cosmétiques ou pharmaceutiques. On citera par exemple la turbine Moritz.
On peut également préparer la composition selon l'invention à l'aide d'un ou plusieurs extrudeurs, ce qui permet notamment d'obtenir une composition comprenant un taux élevé de cires et se présentant sous forme de pâte souple.

Les compositions selon l'invention trouvent une application notamment dans le domaine du maquillage de la peau, des semi-muqueuses, des muqueuses et/ou des phanères, et se présentent alors par exemple sous la forme d'un fond de teint, d'un fard à joues ou à paupières, d'un rouge à lèvres, d'un mascara ou d'un eye-liner.
Elles peuvent également se présenter sous forme non colorée, contenant éventuellement des actifs cosmétiques. Elles peuvent alors notamment être utilisées comme base de soin pour les lèvres ou comme base fixante à appliquer sur un rouge à lèvres classique. La base fixante forme alors un film protecteur sur le film de rouge, en limite le transfert et la migration, et permet d'augmenter ainsi sa tenue.
Les compositions selon l'invention peuvent également se présenter sous la forme d'un produit de soin de la peau, des muqueuses, des semi-muqueuses et/ou des phanères, d'un produit hygiénique ou pharmaceutique ou encore d'un produit solaire ou autobronzant.

L'invention est illustrée plus en détail dans les exemples suivants.

### Exemple 1

On prépare une composition sous forme de bâton de rouge à lèvres ayant la composition suivante :
- huile de silicone phénylée et PDMS 20 %
- tensioactif siliconé 2 %
- glycérine 10 %
- cires (notamment polyéthylène) 17%
- pigments 8 %
- cyclotétradiméthylsiloxane qsp 100%

On chauffe les huiles siliconées, le tensioactif et les cires à 95°C; les pigments sont alors ajoutés. Par ailleurs, on chauffe la glycérine à 80°C; on utilise une turbine MORITZ à la vitesse de rotation de 3000 tr/min pour mélanger la phase grasse et la glycérine jusqu'à l'obtention d'une composition de consistance apte à être coulée; on ajoute alors les composés volatils et l'on coule la composition dans un moule approprié; on laisse refroidir.

On obtient un bâton de rouge à lèvres, de texture agréable et qui s'étale bien et s'applique uniformément. Le film obtenu est très confortable et hydratant pour les lèvres.
Il ne migre pas et ne laisse pas de traces sur des supports extérieurs après évaporation de l'huile volatile (quelques minutes). Ceci est d'autant plus remarquable que la composition comprend une quantité importante de glycérine.

## Revendications

1. Composition cosmétique, dermatologique, hygiénique et/ou pharmaceutique, comprenant un composant hydrophile et une phase grasse qui comprend au moins un composé volatil et au moins une huile siliconée phénylée.

2. Composition cosmétique, dermatologique, hygiénique et/ou pharmaceutique, sans transfert, comprenant un composant hydrophile et une phase grasse qui comprend au moins un composé volatil et au moins une huile siliconée phénylée.

3. Composition selon l'une des revendications précédentes, dans laquelle le composant hydrophile est choisi parmi un alcool polyhydrique et/ou une phase aqueuse épaissie ou gélifiée.

4. Composition selon l'une des revendications précédentes, dans laquelle le composant hydrophile comprend au moins un composé ayant 2-8 atomes de carbone et 2-6 fonctions hydroxyles, tel que l'éthylène glycol, le glycérol, le propane-1,2 diol, la diglycérine, l'érythritol, l'arabitol, l'adonitol, le sorbitol et le dulcitol; et/ou un polyéther alcool de poids moléculaire moyen de 150-600, tel que le polyéthylène glycol 300 et la polyglycérine 500.

5. Composition selon la revendication 3, dans laquelle la phase aqueuse est épaissie à l'aide d'un agent épaississant choisi parmi les extraits d'algues tels que l'agar-agar, les carraghénanes et les alginates; les extraits de graines tels que les extraits de caroube ou de guar; les extraits de fruits, notamment la pectine; les exsudats de plantes, tels que la gomme arabique, l'adragante, la gomme de karaya et la gomme de ghatty; les dérivés cellulosiques, tels que la carboxyméthylcellulose; les gélifiant d'origine animale tels que la gélatine ou les caseinates; les exsudats de micro-organismes tels que la gomme xanthane; les gélifiants synthétiques tels que les dérivés de polymère acrylique ou les dérivés du silicium.

6. Composition selon la revendication 5, dans laquelle l'agent épaississant est présent dans la phase aqueuse à raison de 0,2-10% en poids par rapport à la phase aqueuse.

7. Composition selon l'une des revendications précédentes, dans laquelle le composant hydrophile représente 4 à 40% en poids de la composition, de préférence 8 à 25% en poids.

8. Composition selon l'une des revendications précédentes, comprenant en outre au moins un agent tensioactif.

9. Composition selon l'une des revendications précédentes, dans laquelle le composé volatil est choisi parmi les huiles hydrocarbonées et/ou les huiles siliconées, cycliques ou linéaires, seules ou en mélange.

10. Composition selon l'une des revendications précédentes, dans laquelle le composé volatil est choisi parmi les silicones volatiles cycliques ayant de 3 à 8 atomes de silicium, de préférence de 4 à 6; les cyclocopolymères du type diméthylsiloxane/méthylalkylsiloxane; les silicones volatiles linéaires ayant de 2 à 9 atomes de silicium; les alkyltrisiloxanes; les huiles hydrocarbonées volatiles; et leurs mélanges.

11. Composition selon l'une des revendications précédentes, dans laquelle le composé volatil représente 8-70% en poids, de préférence 15-55%, par rapport au poids total de la composition.

12. Composition selon l'une des revendications précédentes, dans laquelle l'huile de silicone phénylée répond à la formule suivante : 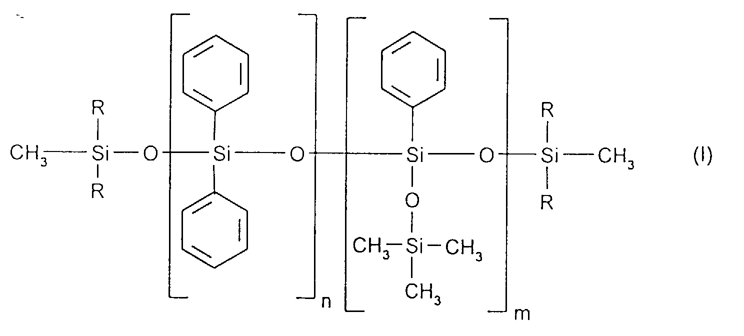 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100.

13. Composition selon l'une des revendications précédentes, dans laquelle l'huile siliconée phénylée représente 1-35% en poids, de préférence 20-30%, par rapport au poids total de la composition.

14. Composition selon l'une des revendications précédentes, comprenant en outre au moins une cire.

15. Composition selon la revendication 14, dans laquelle la cire est choisie parmi les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; les cires de silicone; les cires fluorées; et leurs mélanges.

16. Composition selon l'une des revendications 14 à 15, dans laquelle la cire est présente à raison de 0,5 à 30% en poids, de préférence 10 à 20% en poids, par rapport au poids total de la composition.

17. Composition selon l'une des revendications précédentes, comprenant en outre un corps gras choisi parmi les huiles, les gommes et/ou les corps gras pâteux d'origine végétale, minérale, animale, synthétique ou siliconée.

18. Composition selon l'une des revendications précédentes, comprenant moins de 20% en poids d'huile hydrocarbonée non volatile, de préférence moins de 5% en poids, voire pas d'huiles hydrocarbonées non volatiles du tout.

19. Composition selon l'une des revendications précédentes, comprenant en outre une phase particulaire comprenant des pigments et/ou des charges et/ou des nacres.

20. Composition selon l'une des revendications précédentes, comprenant en outre un agent actif contre les micro-organismes; un agent à activité anti-inflammatoire ou immunomodulatrice; un agent antagoniste des neuromédiateurs ou modulant le relarguage des neuromédiateurs; un agent modulant la différenciation et/ou la prolifération cellulaire et/ou la pigmentation et/ou régulant la kératinisation; un agent actif dans le traitement et/ou la prévention des cheilites; un antihistaminique; un agent cicatrisant.

21. Composition selon l'une des revendications précédentes, se présentant sous la forme d'un stick ou bâton, sous la forme d'un liquide huileux, éventuellement gélifié, ou sous la forme d'une pâte souple de viscosité dynamique à 25°C comprise entre 3 et 35 Pa.s, mesurée avec un viscosimètre rotatif CONTRAVES TV équipé d'un mobile "MS-r4" à la fréquence de 60 Hz.

22. Composition selon l'une des revendications précédentes, se présentant sous la forme d'un produit de soin et/ou de maquillage de la peau, des muqueuses, des semi-muqueuses et des phanères; d'un produit hygiénique; ou d'un produit pharmaceutique.

23. Composition selon l'une des revendications précédentes, se présentant sous la forme d'un fond de teint, d'un fard à joues ou à paupières, d'un rouge à lèvres, d'un mascara, d'un eye-liner, d'un soin pour lèvres, d'une base fixante pour lèvres, d'un autobronzant, d'un produit solaire.

24. Utilisation de l'association d'un composé volatil et d'une huile siliconée phénylée afin de limiter, diminuer et/ou supprimer le transfert et/ou la migration, et/ou afin d'améliorer la tenue, d'une composition cosmétique, dermatologique, hygiénique et/ou pharmaceutique comprenant un composant hydrophile.

25. Procédé pour limiter, diminuer et/ou supprimer le transfert d'une composition cosmétique, dermatologique, hygiénique et/ou pharmaceutique comprenant un composant hydrophile, notamment une composition de maquillage et/ou de soin de la peau, des muqueuses, des semi-muqueuses et/ou des phanères, consistant à introduire dans ladite composition au moins un composé volatil et au moins une huile siliconée phénylée.
